# FASCICULE DE BREVET EUROPEEN

(11) **EP 1 897 509 B1**
(45) Date de publication et mention de la délivrance du brevet: **08.07.2009**
(21) Numéro de dépôt: 07356119.3
(22) Date de dépôt: 10.09.2007
(51) Int. Cl.: A61B 17/80

(54) **Plaque d'arthrodese d'une articulation metatarso-phalangienne**
Arthrodeseplatte für ein Zehengrundgelenk
Arthrodesis plate for a metatarsal-phalanges joint

(30) Priorité: 11.09.2006 FR 0607921
(43) Date de publication de la demande: 12.03.2008
(73) Titulaire: Surge Foot, 01000 Bourg en Bresse (FR)
(72) Inventeur: Arnould, Hervé Charles Marie, 01000 Bourg en Bresse (FR)
(74) Mandataire: Grand, Guillaume

(56) Documents cités:
- EP-A- 1 452 145
- EP-A- 1 616 528
- EP-A1- 1 297 793

## Description

La présente invention concerne une plaque d'arthrodèse d'une articulation métatarso-phalangienne, notamment pour l'articulation entre le premier métatarsien et la première phalange du gros orteil. L'invention concerne également une méthode chirurgicale de pose d'une telle plaque d'arthrodèse.

Lorsqu'une articulation métatarso-phalangienne souffre d'arthrose ou de déformations, dans certains cas iatrogènes, il est fréquent de fusionner le métatarsien et la phalange reliés par cette articulation, c'est-à-dire de bloquer définitivement cette articulation. A cet effet, on utilise généralement une plaque d'arthrodèse, se présentant sous la forme d'un corps allongé globalement plan, mis en place contre les faces supérieures du métatarsien et de la phalange, à cheval sur l'articulation à bloquer. La partie métatarsienne et la partie phalangienne de ce corps comportent respectivement un ou plusieurs trous traversants, dans chacun desquels on introduit, selon une direction globalement verticale, une vis d'ancrage osseux dans, respectivement, le métatarsien et la phalange. Un exemple de ce genre de plaque est donné dans EP-A-1 297 793.

Ce genre de plaque présente plusieurs inconvénients. En particulier, juste avant d'immobiliser définitivement la plaque contre le métatarsien et la phalange, il est généralement nécessaire de rapprocher ces deux os l'un de l'autre, de manière à les rendre sensiblement jointifs au niveau de leur extrémité en regard, préalablement réséquée. Les gestes chirurgicaux correspondants sont délicats, dans le sens où, tout en maintenant jointifs le métatarsien et la phalange, le chirurgien doit visser fermement à la fois une vis dans la partie métatarsienne du corps de plaque et une autre vis dans la partie phalangienne. Les risques d'un positionnement non optimal des deux os à fusionner sont réels, impliquant une éventuelle gêne ultérieure pour le patient. Par ailleurs, la fixation de la plaque tend, à la longue, à s'altérer : lorsque le patient marche, ses articulations métatarso-phalangiennes sont soumises à un mouvement de flexion lié à l'appui progressif de sa voûte plantaire, depuis le talon jusqu'aux orteils. Pour l'articulation bloquée par la plaque, la contrainte de flexion est essentiellement encaissée par cette plaque, ce qui, par répétition cyclique de cette contrainte, fragilise l'ancrage osseux des vis retenant la plaque contre les os fusionnés.

Le but de la présente invention vise à remédier à ces inconvénients, en proposant une plaque d'arthrodèse dont la fixation est simple et pérenne.

A cet effet, l'invention a pour objet une plaque d'arthrodèse d'une articulation métatarso-phalangienne, telle que définie à la revendication 1.

La patte de la plaque selon l'invention permet de fixer la plaque sur une face latérale de l'épiphyse de la phalange, c'est-à-dire, anatomiquement parlant, sur la face médiale de la base phalangienne. Cette zone épiphysaire de l'os est généralement solide, permettant la mise en place d'un moyen d'ancrage s'enfonçant profondément dans la matière osseuse. En pratique, lorsque la plaque selon l'invention est implantée au niveau de l'articulation reliant le premier métatarsien et la première phalange du gros orteil, cette patte est fixée à la face interne de l'épiphyse phalangienne, c'est-à-dire la face osseuse dirigée vers le plan sagittal médian du patient, notamment car cette face présente un abord chirurgical aisé. De plus, cette patte est conformée pour que son trou d'extrémité puisse recevoir une vis longue, ou plus généralement un moyen allongé d'ancrage osseux, qui va s'étendre à la fois à travers la matière osseuse de la phalange et dans la matière osseuse du métatarsien, en particulier dans les épiphyses en regard de ces os. On comprend que cette vis longue s'étend en longueur dans une direction présentant une composante antéro-postérieure, de sorte que cette vis encaisse essentiellement, voire exclusivement les contraintes de flexion générées lors de la marche du patient, étant remarqué que, en raison de sa position, la vis travaille principalement en traction. Comme cette vis présente, de par sa structure et sa zone d'implantation, une capacité à s'opposer aux contraintes de flexion nettement plus élevée que celle du corps de la plaque, l'implantation de la plaque est stable dans le temps. Par ailleurs, lors de l'introduction de la vis longue dans le trou d'extrémité de la patte, cette vis tend à générer d'elle-même un effet de rappel du métatarsien vers la phalange lorsque la pointe de la vis atteint et progresse dans la matière osseuse du métatarsien. D'un seul geste, consistant à visser intégralement cette vis longue, le chirurgien rapproche ainsi automatiquement les deux os à fusionner l'un de l'autre, puis il immobilise totalement le corps de plaque sur le métatarsien pour bloquer l'articulation. On comprend que les deux os rendus ainsi jointifs sont fixés l'un par rapport à l'autre de manière prédéterminée, en fonction de la direction dans laquelle le chirurgien introduit la vis longue dans le trou d'extrémité de la patte conforme à l'invention. Ce trou aide donc le chirurgien, dans le sens où il lui fixe le point d'application de la vis, tout en limitant le risque que la pointe de cette vis s'en écarte, notamment par ripement contre la face latérale de la phalange.

D'autres caractéristiques avantageuses de cette plaque d'arthrodèse, prises isolément ou suivant toutes les combinaisons techniquement possibles, sont énoncées aux revendications 2 à 10.

L'invention a également pour objet une méthode de pose d'une plaque d'arthrodèse d'une articulation métatarso-phalangienne, laquelle plaque comporte un corps de plaque allongé muni d'une patte allongée qui s'étend en longueur depuis un côté longitudinal du corps de plaque et qui est pourvue, à son extrémité longitudinale opposée au corps de plaque, d'un trou traversant, ladite méthode comprenant les gestes chirurgicaux successifs consistant à :
- inciser et dégager des parties molles entourant l'articulation à bloquer par arthrodèse,
- mettre en place le corps de plaque sur les faces supérieures du métatarsien et de la phalange reliés par l'articulation, de manière que le trou de la patte débouche sur une face latérale de l'épiphyse de la phalange,
- immobiliser partiellement le corps de plaque sur le métatarsien, en autorisant une liberté de mouvements relatifs suivant globalement la direction longitudinale du corps de plaque,
- introduire un moyen allongé d'ancrage osseux dans le trou de la patte, tel qu'une vis longue, et ancrer ce moyen dans successivement l'épiphyse de la phalange et le métatarsien, jusqu'à ce qu'une tête d'extrémité du moyen d'ancrage vienne buter contre la patte, au niveau du bord de son trou,
- poursuivre l'ancrage du moyen d'ancrage de manière à provoquer le rapprochement du métatarsien de la phalange, par un déplacement du métatarsien par rapport au corps de plaque suivant globalement la direction longitudinale de ce corps de plaque,
- immobiliser définitivement le corps de plaque sur le métatarsien et la phalange, et
- fermer l'incision.

Suivant une caractéristique avantageuse de cette méthode, on fixe le corps de plaque sur la face supérieure de la phalange soit avant, soit après avoir immobilisé partiellement le corps de plaque sur le métatarsien.

L'invention sera mieux comprise à la lecture de la description qui va suivre, donnée uniquement à titre d'exemple et faite en se référant aux dessins sur lesquels :
- la figure 1 est une vue en perspective d'une plaque d'arthrodèse selon l'invention, mise en place et fixée sur une articulation métatarso-phalangienne bloquée par la plaque ;
- la figure 2 est une vue de la plaque de la figure 1, observée selon la flèche II de la figure 1, qui correspond à une direction d'observation sensiblement verticale ;
- les figures 3 à 6 sont des vues en élévation, prises respectivement selon les flèches III à VI de la figure 2 ; et
- la figure 7 est une coupe partielle de la plaque, selon la ligne VII-VII de la figure 2.

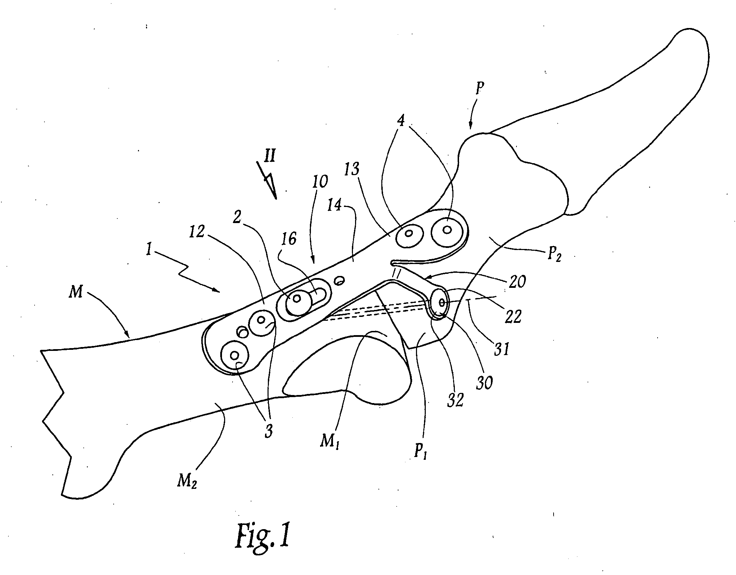

Sur la figure 1 est représentée une plaque 1 d'arthrodèse d'une articulation entre le premier métatarsien M et la première phalange P du gros orteil d'un pied gauche. Par commodité, la suite de la description est orientée par rapport au patient dont les os du gros orteil sont représentés à la figure 1, ce patient se tenant debout sur un sol horizontal. Ainsi, les termes « inférieur » et « bas » désignent une direction sensiblement verticale dirigée vers le sol, tandis que les termes « supérieur » et « haut » désignent une direction de sens opposé. De même, le terme « intérieur » désigne une direction sensiblement horizontale dirigée vers le plan sagittal médian du patient, tandis que le terme « extérieur » désigne une direction de sens opposé. Les termes « avant », « arrière » et analogues s'entendent également par rapport au patient.

En pratique, la plaque 1 considérée aux figures appartient avantageusement à un jeu de plusieurs plaques d'arthrodèse, ayant des tailles respectives sensiblement homothétiques. La plaque 1 représentée est la plus appropriée au patient, notamment à la taille de son métatarsien M et de sa phalange P.

La plaque 1 comporte essentiellement, d'une part, un corps de plaque 10 présentant une forme allongée suivant une direction globalement antéro-postérieure et, d'autre part, une patte allongée intérieure 20.

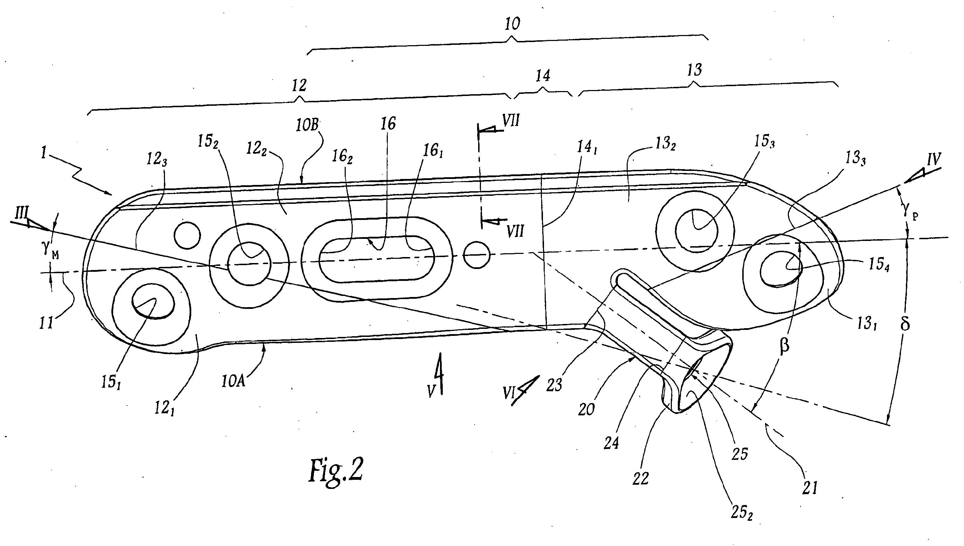

Comme représenté plus en détail sur les figures 2 à 7, dans la configuration d'implantation de la plaque 1, le corps de plaque 10 inclut successivement, suivant sa direction longitudinale 11, une partie métatarsienne 12 et une partie phalangienne 13, qui sont respectivement adaptées pour être mises en place et fixées sur le métatarsien M et la phalange P, avec leur face inférieure plaquée contre, respectivement, les surfaces supérieures du métatarsien et de la phalange. La zone 14 de jonction entre les parties 12 et 13 est prévue pour surplomber la zone de jointement entre les extrémités épiphysaires en regard M₁ et P₁ du métatarsien M et de la phalange P, de sorte que le corps de plaque 10 s'étend à cheval sur l'articulation métatarso-phalangienne selon la direction 11.

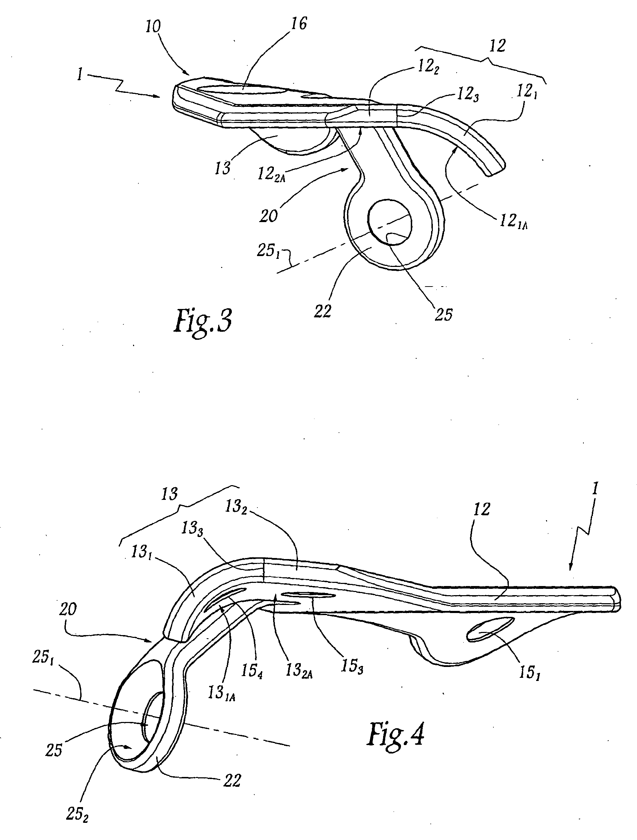

Sur le côté longitudinal intérieur 10A du corps de plaque 10, la partie métatarsienne 12 comprend une section cintrée 12₁ reliée à la zone de jonction 14 par une section sensiblement plane 12₂ de cette partie 12. Ces sections 12₁ et 12₂ sont pliées l'une par rapport à l'autre le long d'une ligne de pliage 12₃ s'étendant dans le prolongement rectiligne de la flèche III. La surface inférieure concave 12_{1A} de la section cintrée 12₁, bien visible à la figure 3, est conformée pour épouser la face supérieure de la partie diaphysaire M₂ du métatarsien M, c'est-à-dire pour venir au contact de cette diaphyse, tout en la recouvrant partiellement de manière ajustée, comme représenté sur la figure 1. La surface inférieure sensiblement plate 12_{2A} de la section plane 12₂ est prévue pour recouvrir la zone dorsale de la diaphyse métatarsienne M₂ et, surtout, l'épiphyse métatarsienne M₁ qui a été réséquée préalablement à la mise en place de la plaque. Autrement dit, on comprend que la surface 12_{2A} est prévue pour être mise en place contre une zone surfacique globalement plate de la face supérieure du métatarsien M, tandis que la surface 12_{1A} recouvre une zone métatarsienne bombée.

Eu égard à la forme anatomique du métatarsien M, la ligne de pliage 12₃ forme, selon la direction d'observation de la flèche II, un angle γ_{M} non nul avec la direction longitudinale 11 du corps de plaque 10. Avantageusement, pour permettre un bon ajustement des surfaces inférieures 12_{1A} et 12_{2A} avec la face supérieure du métatarsien, cet angle γ_{M} est compris entre 10 et 20°, étant remarqué que des valeurs homothétiques de γ_{M} sont avantageusement prévues entre les bornes 12° et 18° pour le jeu des plaques de tailles respectives différentes.

Également sur le côté longitudinal intérieur 10A du corps de plaque 10, la partie phalangienne 13 comprend une section cintrée 13₁ reliée à la zone de jonction 14 par une section sensiblement plane 13₂ de cette partie 13. Ces sections 13₁ et 13₂ sont pliées l'une par rapport à l'autre selon une ligne de pliage 13₃ qui s'étend dans le prolongement rectiligne de la flèche IV. Par analogie avec les explications précédentes concernant les sections 12₁ et 12₂ de la partie métatarsienne 12, on comprend que la surface inférieure concave 13_{1A} de la section cintrée 13₁, bien visible à la figure 4, est dimensionnée pour venir épouser la face médiale bombée de la diaphyse phalangienne P₂, tandis que la surface inférieure 13_{2A} de la section 13₂ recouvre la face dorsale de cette diaphyse et, surtout, l'épiphyse phalangienne P₁, comme représenté à la figure 1.

Pour des raisons anatomiques, la ligne de pliage 13₃ est, selon la direction d'observation correspondant à la flèche II, inclinée par rapport à la direction 11, en formant avantageusement avec cette dernière un angle non nul γ_{P} compris entre 15 et 25°. De préférence, l'angle γ_{P} est égal à 20° +/- 1°.

Selon la direction d'observation correspondant à la flèche II, les lignes de pliage 12₃ et 13₃ forment globalement un V dont la pointe est dirigée vers l'intérieur.

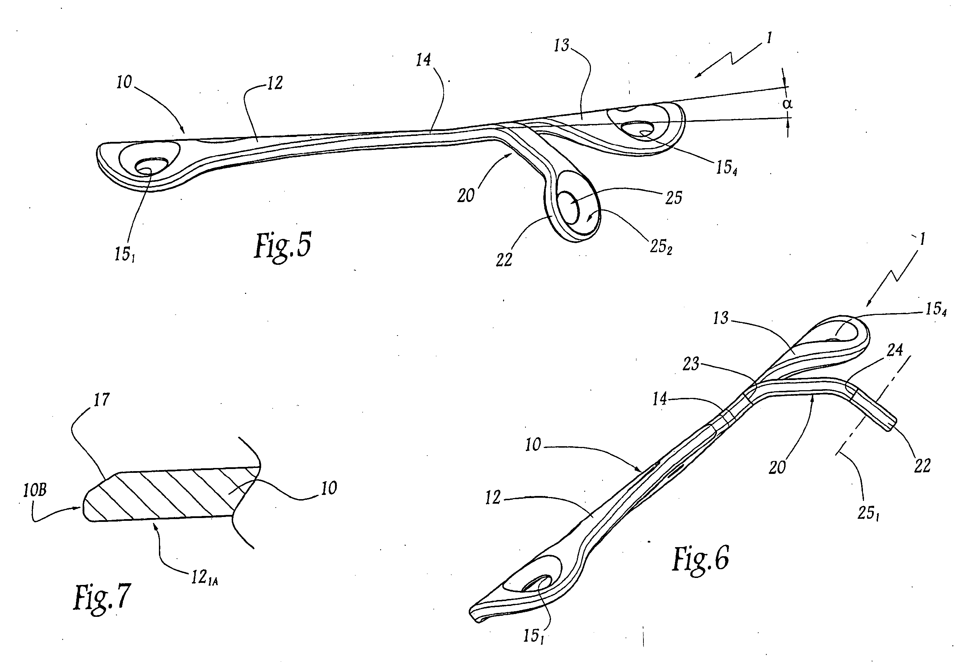

Selon une direction d'observation médio-latérale, comme celle correspondant à la flèche V, on note que les sections planes métatarsienne 12₂ et phalangienne 13₂ ne s'étendent pas dans le prolongement rectiligne l'une de l'autre, mais forment au contraire un angle non nul, noté α à la figure 5. A cet effet, les parties métatarsienne 12 et phalangienne 13 sont pliées l'une par rapport à l'autre au niveau de leur zone de jonction 14, selon une ligne de pliage 14₁ sensiblement perpendiculaire à la direction longitudinale 11. Grâce à cette disposition, la partie phalangienne 13 est inclinée vers le haut dans un plan vertical par rapport à la partie métatarsienne 12, ce qui garantit une meilleure adaptation du corps de plaque 10 à l'anatomie de l'articulation métatarso-phalangienne lorsqu'elle est bloquée. En effet, la phalange P est alors immobilisée par rapport au métatarsien M avec un angle de dorsi-flexion non nul, correspondant à l'angle α.

Pour permettre la fixation du corps de plaque 10 au métatarsien M et à la phalange P, ce corps est pourvu d'une série de trous traversants, adaptés chacun pour recevoir de manière complémentaire une vis d'ancrage osseux ou un moyen mécanique analogue. Avantageusement, chacune des sections 12₁, 12₂, 13₁ et 13₂ est munie d'au moins un de ces trous, respectivement référencés 15₁, 15₂, 15₃ et 15₄. De la sorte, lors du vissage de vis dans ces trous, les surfaces inférieures respectives 12_{1A}, 12_{2A}, 13_{1A} et 13_{2A} de ces sections sont fermement appliquées contre les os.

La partie métatarsienne 12 est en outre munie d'un orifice traversant 16 de forme oblongue, dont la direction longitudinale est sensiblement parallèle à la direction longitudinale 11 du corps de plaque 10. L'intérêt de cet orifice oblong sera mis en évidence plus loin, lors de la description de la pose de la plaque d'arthrodèse 1.

La patte 20, également située du côté longitudinal intérieur 10A du corps de plaque 10, s'étend en longueur depuis la partie phalangienne 13. Comme représenté à la figure 1, cette patte est conformée pour envelopper l'épiphyse phalangienne P₁, au plus proche de la matière osseuse, incluant son extrémité libre 22, c'est-à-dire son extrémité longitudinale opposée au corps de plaque 10. La patte 20 donne ainsi l'impression de plonger vers le bas par rapport au corps de plaque 10, de manière que son extrémité 22, située verticalement au-dessous de ce corps de plaque dans la configuration d'implantation de la plaque 1, soit plaquée contre la face latérale intérieure de l'épiphyse phalangienne P₁.

A cet effet, la patte 20 est pliée vers le bas par rapport au corps de plaque 10 le long d'une ligne de pliage 23 sensiblement perpendiculaire à la direction longitudinale 21 et située au niveau de la jonction entre la patte et la partie phalangienne 13. La patte 20 est également pliée sur elle-même selon une autre ligne de pliage 24 sensiblement perpendiculaire à la direction 21 et située à la jonction entre son extrémité 22 et le reste de la plaque. La forme pliée de la patte 20 est bien visible à la figure 6, dont la direction d'observation, qui correspond à la flèche VI de la figure 2, est alignée avec la ligne de pliage 23.

Selon la direction d'observation correspondant à la flèche II, la patte 20 est inclinée par rapport au corps de plaque 10 : sa direction longitudinale 21 forme un angle non nul β avec la direction longitudinale 11 du corps de plaque, comme indiqué à la figure 2. Pour des raisons anatomiques, cet angle β est compris entre 20 et 60°. De préférence, l'angle β est égal à 40° +/- 1 °, ce qui permet de disposer de la patte 20 sans que cette dernière ne limite trop l'étendue transversale de la partie phalangienne 13, tout en enveloppant au plus près l'épiphyse phalangienne P₁.

A son extrémité 22, la patte 20 est pourvue d'un trou traversant 25 adapté pour recevoir une vis 30 ou, plus généralement, un moyen allongé d'ancrage osseux analogue. Cette vis 30 est une vis longue dans le sens où, comme représenté en pointillés à la figure 1, elle présente une longueur suffisante pour s'étendre depuis le trou 25 à la fois dans l'épiphyse phalangienne P₁ et dans l'épiphyse métatarsienne M₁, voire également dans la diaphyse métatarsienne M₂.

Le trou 25 est prévu pour recevoir la vis 30 de sorte que, selon la direction d'observation correspondant à la flèche II, l'axe longitudinal 31 de cette vis puisse être incliné par rapport à la direction longitudinale 11 du corps de plaque 10, en formant avec cette direction 11 un angle non nul δ. On comprend que plus cet angle δ est petit, plus l'axe 31 de la vis 30 tend à s'aligner avec une direction antéro-postérieure, garantissant une profondeur de pénétration de la vis dans le métatarsien plus importante, à longueur de vis donnée. En pratique, pour permettre de visser et de bloquer la vis 30 avec son axe 31 incliné par rapport à l'axe central 25₁ du trou 25, le bord 25₂ de ce trou présente, du côté tourné vers la tête 32 de la vis, une surface concave, sensiblement complémentaire d'une surface associée délimitée par cette tête de vis. De la sorte, lorsque la vis 31 est totalement introduite dans le trou 25, sa tête 32 vient prendre appui et se coincer contre au moins une portion du bord 25₂, même si son axe 31 est incliné par rapport à l'axe 25₁ du trou.

Pour des raisons anatomiques, l'angle δ est avantageusement choisi inférieur à 45°. Dans l'exemple considéré aux figures, cet angle vaut environ 20°.

Sur son côté longitudinal extérieur 10B, le corps de plaque 10 est chanfreiné, comme représenté à la figure 7. Le chanfrein correspondant 17, globalement plat, est tourné vers le haut dans la configuration d'implantation de la plaque 1. De la sorte, lorsque la plaque est implantée, les risques que cette dernière gêne, voire ne presse significativement le tendon extenseur de l'articulation métatarso-phalangienne sont limités.

Une méthode de pose de la plaque d'arthrodèse 1 est la suivante. Après avoir incisé et dégagé les parties molles entourant l'articulation à bloquer, le chirurgien résèque en partie les épiphyses métatarsienne M₁ et phalangienne P₁, afin de supprimer des irrégularités osseuses telles que des renflements d'arthrose présents dans l'articulation.

Après avoir choisi, parmi le jeu de plusieurs plaques d'arthrodèse homothétiques, la plaque 1 dont la taille est la plus appropriée au patient opéré, le chirurgien met ensuite en place la plaque sur les os M et P : le corps de plaque 10 est placé sur les faces supérieures de ces os, tandis que l'extrémité 22 de la patte 20 est placée contre la face latérale intérieure de l'épiphyse phalangienne P₁, c'est-à-dire, anatomiquement, sa face médiale. Le corps de plaque 10 est ensuite partiellement immobilisé en utilisant l'orifice oblong 16 : le chirurgien introduit une vis 2 (figure 1) dans l'orifice 16, du côté du fond arrière 16₂ de cet orifice, sans serrer la tête de vis contre le bord de l'orifice. De la sorte, le corps de plaque 10 reste déplaçable selon la direction 11 par rapport au métatarsien M.

La vis 30 est ensuite introduite dans le trou 25, suivant une direction d'introduction inclinée par rapport au corps de plaque 10 sous l'angle δ dont la valeur est choisie par le chirurgien pour que cette vis, lors de son vissage, traverse successivement l'épiphyse phalangienne P₁ et l'épiphyse métatarsienne M₁, comme expliqué plus haut. Le vissage de la vis 30 est poursuivi jusqu'à ce que la tête 32 de cette vis vienne buter, selon son axe 31, contre le bord 25₂ du trou 25. En poursuivant le vissage, la vis 30 provoque le rapprochement du métatarsien M par rapport à la phalange P, ce mouvement de rapprochement étant guidé le long de la direction 11 par la coopération de l'orifice oblong 16 et de la vis non serrée 2. Le fond avant 16₁ de l'orifice 16 progresse alors vers l'arrière vis-à-vis de la vis 2, qui se retrouve alors progressivement du côté de ce fond avant 16₁. Le chirurgien poursuit ainsi le vissage de la vis 30 jusqu'à rendre sensiblement jointifs les extrémités épiphysaires M₁ et P₁ des os. On comprend que la direction d'introduction de la vis 30 dans le trou 25 conditionne le positionnement relatif du métatarsien et de la phalange à leur fusion.

La vis 2 est ensuite totalement vissée et serrée dans l'orifice 16, de manière à immobiliser complètement le corps de plaque 10 sur le métatarsien M. Cette fixation est renforcée en vissant des vis 3 dans les trous 15₁ et 15₂.

Avant ou après avoir ainsi immobilisé le corps de plaque 10 vis-à-vis du métatarsien M, d'autres vis 4 sont introduites dans les trous 15₃ et 15₄, de manière à fixer la partie phalangienne 13 sur la phalange P.

Le chirurgien ferme enfin l'incision.

En pratique, le chirurgien dispose bien entendu de plaques d'arthrodèse pour articulation métatarso-phalangienne du pied droit : ces plaques présentent une structure symétrique à celle de la plaque 1 par rapport à un plan vertical médian à cette dernière.

Avantageusement, pour faciliter le choix de la taille de la plaque à implanter, le chirurgien dispose en outre d'un jeu de fantômes de plaque d'arthrodèse : chaque fantôme présente des contours extérieurs identiques à ceux d'une des plaques d'arthrodèse disponibles dans le jeu à sa disposition, de sorte que, après avoir incisé le patient et effectué des premières résections osseuses, le chirurgien met en place l'un de ces fantômes, d'une part, pour sélectionner la taille d'implant la plus appropriée et, d'autre part, pour s'assurer que les résections sont suffisantes, notamment au niveau de la zone de coaptation entre plaque d'une part et métatarsien et phalange d'autre part.

Par ailleurs, lors de la mise en place de la plaque 1, le chirurgien a avantageusement la possibilité de modifier l'angle α pour ajuster au mieux cet angle avec l'angle anatomique de dorsi-flexion entre le métatarsien M et la phalange P du patient opéré, en tenant compte notamment des résections osseuses et de la morphologie générale des os du patient, ainsi que des habitudes de chaussage et plus particulièrement la hauteur du talon de la chaussure.

La fabrication de la plaque 1 est réalisée à partir d'une plaque plane métallique. Cette plaque est, dans un premier temps, découpée suivant des contours prédéterminés afin d'obtenir les parties métatarsienne 12 et phalangienne 13, ainsi que la patte 20. Dans un second temps, les différents trous 15₁ à 15₄ et l'orifice oblong 16 sont percés. Dans un troisième temps, les sections 12₁ et 13₁ sont cintrées, tandis que la plaque est pliée le long des lignes 12₃, 13₃, 23 et 24.

Divers aménagements et variantes à la plaque 1 décrite ci-dessus, ainsi qu'à sa méthode de pose sont envisageables. A titre d'exemples :
- la face inférieure de la plaque est grenaillée ou, de manière plus générale, subit un traitement de surface, afin de favoriser une colonisation osseuse une fois que la plaque est implantée ; et/ou
- le nombre de trous 15₁ à 15₄ peut être modifié, notamment en fonction de la taille longitudinale du corps de plaque 10.

## Revendications

1. Plaque (1) d'arthrodèse d'une articulation métatarso-phalangienne, comportant un corps de plaque allongé (10) adapté pour être mis en place et fixé sur les faces supérieures respectives du métatarsien (M) et de la phalange (P) reliés par l'articulation à bloquer par arthrodèse, de manière à s'étendre en longueur à cheval sur l'articulation,
**caractérisée en ce que** le corps de plaque (10) est muni d'une patte allongée (20) de fixation à une face latérale de l'épiphyse (P₁) de la phalange (P), laquelle patte s'étend en longueur depuis un côté longitudinal (10A) du corps de plaque et est pourvue, à son extrémité longitudinale (22) opposée au corps de plaque, d'un trou traversant (25) adapté pour recevoir un moyen allongé (30) d'ancrage osseux dans, à la fois, la phalange (P) et le métatarsien (M), tel qu'une vis longue.

2. Plaque selon la revendication 1, **caractérisée en ce que**, selon une direction d'observation (flèche II) sensiblement verticale lorsque la plaque (1) est implantée, la patte (20) s'étend suivant une direction longitudinale (21) qui forme avec la direction longitudinale (11) du corps de plaque (10) un angle (β) compris entre 20 et 60°, de préférence égal à 40° +/- 1°.

3. Plaque selon l'une des revendications 1 ou 2, **caractérisée en ce que** le trou traversant (25) de la patte (20) est adapté pour recevoir le moyen d'ancrage osseux (30) dans une position dans laquelle, selon une direction d'observation (flèche II) sensiblement verticale lorsque la plaque (1) est implantée, la direction longitudinale (31) de ce moyen d'ancrage, tel que l'axe central d'une vis longue, forme avec la direction longitudinale (11) du corps de plaque (10) un angle (δ) inférieur à 45°.

4. Plaque selon l'une quelconque des revendications précédentes, **caractérisée en ce que** la patte (20) est pliée selon au moins une ligne de pliage (23, 24) sensiblement perpendiculaire à la direction longitudinale (21) de la patte.

5. Plaque selon l'une quelconque des revendications précédentes, **caractérisée en ce que** le corps de plaque (10) comprend, suivant sa longueur, une partie métatarsienne (12) et une partie phalangienne (13), respectivement adaptées pour être mises en place et fixées sur les faces supérieures du métatarsien (M) et de la phalange (P), et **en ce que** la patte (20) s'étend depuis la partie phalangienne (13), en étant de préférence monobloc avec cette dernière.

6. Plaque selon la revendication 5, **caractérisée en ce que** la partie métatarsienne (12) est pourvue d'un orifice traversant oblong (16) dont la direction longitudinale est sensiblement parallèle à la direction longitudinale (11) du corps de plaque (10).

7. Plaque selon l'une des revendications 5 ou 6, **caractérisée en ce que** la, partie métatarsienne (12) inclut, d'une part, du même côté longitudinal (10A) du corps de plaque (10) que la patte (20), une section cintrée (12₁), dont la face concave (12_{1A}) est adaptée pour venir au contact et pour envelopper la face supérieure bombée de la diaphyse métatarsienne (M₂), et, d'autre part, une section sensiblement plane (12₂) reliant la section cintrée à la partie phalangienne (13).

8. Plaque selon la revendication 7, **caractérisée en ce que** la section cintrée (12₁) et la section sensiblement plane (12₂) de la partie métatarsienne (12) sont pliées l'une par rapport à l'autre selon une ligne de pliage (12₃) qui, selon une direction d'observation (flèche II) sensiblement verticale lorsque la plaque (1) est implantée, forme avec la direction longitudinale (11) du corps de plaque (10) un angle (γ_{M}) compris entre 10 et 20°, des valeurs homothétiques de cet angle étant de préférence prévues entre les bornes 12° et 18° pour un jeu de plaques d'arthrodèse présentant des tailles respectives différentes.

9. Plaque selon l'une quelconque des revendications 5 à 8, **caractérisée en ce que** la partie phalangienne (13) inclut, d'une part, du même côté longitudinal (10A) du corps de plaque (10) que la patte (20), une section cintrée (13₁), dont la face concave (13_{1A}) est adaptée pour venir au contact et pour envelopper la face supérieure bombée de la diaphyse phalangienne (P₂), et, d'autre part, une section sensiblement plane (13₂) reliant la section cintrée à la partie métatarsienne (12).

10. Plaque selon la revendication 9, **caractérisée en ce que** la section cintrée (13₁) et la section sensiblement plane (13₂) de la partie phalangienne (13) sont pliées l'une par rapport à l'autre selon une ligne de pliage (13₃) qui, selon une direction d'observation (flèche II) sensiblement verticale lorsque la plaque (1) est implantée, forme avec la direction longitudinale (11) du corps de plaque (10) un angle (γ_{P}) compris entre 15 et 25°, de préférence égal à 20° +/-1°.

## Claims

1. Arthrodesis plate (1) for a metatarsophalangeal joint, comprising an elongated plate body (10) adapted to be put in place and fixed on the respective top faces of the metatarsal (M) and phalange (P) connected by the joint to be locked by arthrodesis so as to extend lengthwise straddling the joint,
**characterised in that** the plate body (10) is fitted with an elongated leg (20) for fixing to a lateral face of the epiphysis (P₁) of the phalange (P), which leg extends lengthwise from a longitudinal side (10A) of the plate body and at the longitudinal end (22) opposite the plate body is provided with a through hole (25) adapted to take an elongated means (30) of osseous anchorage in the phalange (P) and metatarsal (M) at the same time, such as a long screw.

2. Plate according to claim 1, **characterised in that** according to a more or less vertical direction of observation (arrow II), when the plate (1) is implanted, the leg (20) extends in a longitudinal direction (21), which forms an angle (β) of between 20 and 60°, preferably equal to 40° +/-1°, with the longitudinal direction (11) of the plate body (10).

3. Plate according to one of claims 1 or 2, **characterised in that** the through hole (25) of the leg (20) is adapted to take the means of osseous anchorage (30) in a position, in which according to a more or less vertical direction of observation (arrow II), when the plate (1) is implanted, the longitudinal direction (31) of this means of anchorage, such as the central shaft of a long screw, forms an angle (δ) of less than 45° with the longitudinal direction (11) of the plate body (10).

4. Plate according to any one of the previous claims, **characterised in that** the leg (20) is folded according to at least one folding line (23, 24) more or less perpendicular to the longitudinal direction (21) of the leg.

5. Plate according to any one of the previous claims, **characterised in that** along its length the plate body (10) comprises a metatarsal part (12) and a phalangeal part (13), adapted to be put in place and fixed on the top faces of the metatarsal (M) and phalange (P) respectively, and **in that** the leg (20) extends from the phalangeal part (13), preferably forming a solid block with it.

6. Plate according to claim 5, **characterised in that** the metatarsal part (12) is provided with an oblong through hole (16), the longitudinal direction of which is more or less parallel to the longitudinal direction (11) of the plate body (10).

7. Plate according to one of claims 5 or 6, **characterised in that** the metatarsal part (12) includes on the one hand, on the same longitudinal side (10A) of the plate body (10) as the leg (20), an arched section (12₁), the concave face (12_{1A}) of which is adapted to come into contact and surround the top convex face of the metatarsal diaphysis (M₂), and on the other hand a more or less flat section (12₂) connecting the arched section to the phalangeal part (13).

8. Plate according to claim 7, **characterised in that** the arched section (12₁) and the more or less flat section (12₂) of the metatarsal part (12) are folded in relation to each other according to a folding line (12₃), which according to a more or less vertical direction of observation (arrow II), when the plate (1) is implanted, forms an angle (γ_{M}) of between 10 and 20° with the longitudinal direction (11) of the plate body (10), homothetical values of this angle preferably being provided between the limits of 12° and 18° for a set of arthrodesis plates of different respective sizes.

9. Plate according to any one of claims 5 to 8, **characterised in that** the phalangeal part (13) includes on the one hand, on the same longitudinal side (10A) of the plate body (10) as the leg (20), an arched section (13₁), the concave face (13_{1A}) of which is adapted to come into contact and surround the top convex face of the phalange diaphysis (P₂), and on the other hand a more or less flat section (13₂) connecting the arched section to the metatarsal part (12).

10. Plate according to claim 9, **characterised in that** the arched section (13₁) and the more or less flat section (13₂) of the phalangeal part (13) are folded in relation to each other according to a folding line (13₃), which according to a more or less vertical direction of observation (arrow II), when the plate (1) is implanted, forms an angle (γ_{P}) of between 15 and 25°, preferably equal to 20° +/-1°, with the longitudinal direction (11) of the plate body (10).

## Patentansprüche

1. Arthrodeseplatte (1) für ein metatarsophalangeales Gelenk, umfassend einen langgestreckten Plattenkörper (10), der dafür eingerichtet ist, an den jeweiligen Oberseiten des Metatarsalknochens (M) und der Phalanx (P), die durch das mittels Arthrodese zu blockierende Gelenk verbunden sind, angebracht und befestigt zu werden, derart, dass er sich in Längsrichtung auf dem Gelenk aufsitzend erstreckt,
**dadurch gekennzeichnet, dass** der Plattenkörper (10) mit einer langgestreckten Lasche (20) zur Befestigung an einer Seitenfläche der Epiphyse (P₁) der Phalanx (P) ausgestattet ist, wobei sich diese Lasche in Längsrichtung von einer Längsseite (10A) des Plattenkörpers aus erstreckt und an ihrem dem Plattenkörper gegenüberliegenden Längsende (22) mit einem Durchgangsloch (25) versehen ist, das dafür eingerichtet ist, ein langgestrecktes Mittel (30) zur Knochenverankerung gleichzeitig in der Phalanx (P) und dem Metatarsalknochen (M) aufzunehmen, wie etwa eine lange Schraube.

2. Platte nach Anspruch 1, **dadurch gekennzeichnet, dass** in einer im Wesentlichen vertikalen Betrachtungsrichtung (Pfeil II), wenn die Platte (1) implantiert ist, die Lasche (20) sich in einer Längsrichtung (21) erstreckt, welche mit der Längsrichtung (11) des Plattenkörpers (10) einen Winkel (β) bildet, der zwischen 20° und 60° beträgt und vorzugsweise gleich 40° +/- 1° ist.

3. Platte nach einem der Ansprüche 1 oder 2, **dadurch gekennzeichnet, dass** das Durchgangsloch (25) der Lasche (20) dafür eingerichtet ist, das Mittel zur Knochenverankerung (30) in einer Position aufzunehmen, in welcher in einer im Wesentlichen vertikalen Betrachtungsrichtung (Pfeil II), wenn die Platte (1) implantiert ist, die Längsrichtung (31) dieses Verankerungsmittels, wie etwa die Mittelachse einer langen Schraube, mit der Längsrichtung (11) des Plattenkörpers (10) einen Winkel (δ) bildet, der kleiner als 45° ist.

4. Platte nach einem der vorhergehenden Ansprüche, **dadurch gekennzeichnet, dass** die Lasche (20) entlang mindestens einer Biegelinie (23, 24) umgebogen ist, die im Wesentlichen senkrecht zur Längsrichtung (21) der Lasche ist.

5. Platte nach einem der vorhergehenden Ansprüche, **dadurch gekennzeichnet, dass** der Plattenkörper (10), in seiner Längsrichtung gesehen, einen metatarsalen Teil (12) und einen phalangealen Teil (13) umfasst, die dafür eingerichtet sind, an der Oberseite des Metatarsalknochens (M) bzw. der Phalanx (P) angebracht und befestigt zu werden, und **dadurch**, dass sich die Lasche (20) von dem phalangealen Teil (13) aus erstreckt, wobei sie vorzugsweise mit Letzterem aus einem Stück besteht.

6. Platte nach Anspruch 5, **dadurch gekennzeichnet, dass** der metatarsale Teil (12) mit einer länglichen durchgehenden Öffnung (16) versehen ist, deren Längsrichtung im Wesentlichen parallel zur Längsrichtung (11) des Plattenkörpers (10) ist.

7. Platte nach einem der Ansprüche 5 oder 6, **dadurch gekennzeichnet, dass** der metatarsale Teil (12) einerseits auf derselben Längsseite (10A) des Plattenkörpers (10), auf der sich die Lasche (20) befindet, einen rundgebogenen Abschnitt (12₁) enthält, dessen konkave Seite (12_{1A}) dafür eingerichtet ist, mit der gewölbten Oberseite der Metatarsalknochendiaphyse (M₂) in Kontakt zu kommen und sie zu umhüllen, und andererseits einen im Wesentlichen ebenen Abschnitt (12₂), der den rundgebogenen Abschnitt mit dem phalangealen Teil (13) verbindet.

8. Platte nach Anspruch 7, **dadurch gekennzeichnet, dass** der rundgebogene Abschnitt (12₁) und der im Wesentlichen ebene Abschnitt (12₂) des metatarsalen Teils (12) relativ zueinander um eine Biegelinie (12₃) umgebogen sind, welche in einer im Wesentlichen vertikalen Betrachtungsrichtung (Pfeil II), wenn die Platte (1) implantiert ist, mit der Längsrichtung (11) des Plattenkörpers (10) einen Winkel (γ_{M}) bildet, der zwischen 10° und 20° beträgt, wobei homothetische Werte dieses Winkels vorzugsweise zwischen den Begrenzungen 12° und 18° für einen Satz von Arthrodeseplatten vorgesehen sind, die unterschiedliche jeweilige Größen aufweisen.

9. Platte nach einem der Ansprüche 5 bis 8, **dadurch gekennzeichnet, dass** der phalangeale Teil (13) einerseits auf derselben Längsseite (10A) des Plattenkörpers (10), auf der sich die Lasche (20) befindet, einen rundgebogenen Abschnitt (13₁) enthält, dessen konkave Seite (13_{1A}) dafür eingerichtet ist, mit der gewölbten Oberseite der Phalanxdiaphyse (P₂) in Kontakt zu kommen und sie zu umhüllen, und andererseits einen im Wesentlichen ebenen Abschnitt (13₂), der den rundgebogenen Abschnitt mit dem metatarsalen Teil (12) verbindet.

10. Platte nach Anspruch 9, **dadurch gekennzeichnet, dass** der rundgebogene Abschnitt (13₁) und der im Wesentlichen ebene Abschnitt (13₂) des phalangealen Teils (13) relativ zueinander um eine Biegelinie (13₃) umgebogen sind, welche in einer im Wesentlichen vertikalen Betrachtungsrichtung (Pfeil II), wenn die Platte (1) implantiert ist, mit der Längsrichtung (11) des Plattenkörpers (10) einen Winkel (γ_{P}) bildet, der zwischen 15° und 25° beträgt und vorzugsweise gleich 20° +/- 1° ist.
